# EUROPÄISCHE PATENTANMELDUNG

(11) **EP 3 659 636 A1**
(43) Veröffentlichungstag der Anmeldung: **03.06.2020**
(21) Anmeldenummer: 19210610.2
(22) Anmeldetag: 21.11.2019
(51) Int. Cl.: A61L 31/04

(54) **BIOINERTER KÖRPER**

(30) Priorität: 28.11.2018 DE 102018130153
(71) Anmelder: Kasperk, Christian, 69123 Heidelberg (DE); Haas, Andreas, 69207 Sandhausen (DE)
(72) Erfinder: HAAS, Andreas, 69207 Sandhausen (DE)
(74) Vertreter: Wunderlich & Heim Patentanwälte Partnerschaftsgesellschaft mbB

(57) **Zusammenfassung**

Die Erfindung betrifft einen bioinerten Körper mit einem Basiskörper, der bei Hydration auf das Mehrfache seines Volumens anwächst. Der Basiskörper besteht im Wesentlichen aus getrockneten Laminariateilen, die durch den Jodgehalt auch eine lokale antbakterielle Wirkung entfalten. Der bioinerte Körper ist zur Anwendung in einem Verfahren der Gewebevermehrung geeignet.

## Beschreibung

Die Erfindung betrifft einen bioinerten Körper mit einem Basiskörper, dessen Volumen veränderbar ist.

Zur Vorbereitung für eine Zahnimplantatversorgung oder für einen besseren Vollprothesensitz ist es oft erforderlich ein Implantatmaterial subperiostal einzubringen, wo es dann ossär einheilen kann. Oft ist jedoch das Knochenangebot, das heißt die vertikale und horizontale Dimension des Kieferknochens, für das Einbringen eines Implantates in den Knochen nicht ausreichend dimensioniert. Dann sind Maßnahmen zum Aufbau des Knochens, sogenannte Knochenaugmentationen oder Sinusbodenelevationen, sofern diese im Bereich der Nasennebenhöhlenregion sattfindet, notwendig.

Die Mundschleimhaut liegt direkt und fest auf dem Knochen auf. Daher kann es bei ausgedehnten Knochenaugmentationen zu einem Mangel an Schleimhaut, also Weichgewebe, kommen, sodass ein spannungsfreier suffizienter Verschluss, der zur Operation notwendigerweise durchtrennten Schleimhaut über dem raumfordernden und empfindlichen eingebrachten Knochenaugmentat nicht mehr möglich ist.

Zwar ist die Schleimheit selbst dehnbar, jedoch ist sie anatomisch der nicht dehnbaren Knochenhaut, dem Periost, direkt aufliegend und mit dieser verwachsen. Die dem Kieferkamm anliegende Schleimhaut und die direkt dem Knochen anliegende Knochenhaut müssen also idealerweise zunächst zusammen mobilisiert und gedehnt werden, damit unter der Mundschleimhaut-Periost-Schicht ein ausreichender Raum geschaffen werden kann, in den das Knochenaugmentat eingebracht werden kann.

In einigen Fällen kann durch sogenannte Periostschlitzung, eine Dehnung der Schleimhaut und somit ein Wundverschluss erreicht werden. Problematisch bei diesem Vorgehen sind dabei jedoch mehrere Punkte.

Zum Wundverschluss ist er erforderlich, dass die Schleimhaut samt Periost vom Knochen weiter gelöst werden als zuvor ohne Augmentat nötig. Damit besteht allerdings die Gefahr, dass dieser Teil der gelösten Schleimhaut sich nicht wieder in gleicher Weise an den Knochen anlegt und die zuvor fest am Knochen anliegende Schleimhaut somit mobil bleibt. Dies kann ungünstig für die geplante Implantation sein und kann im weiteren Behandlungsverlauf weitere Operationen, wie eine Vestibulimplastik oder eine Mundbodensenkung erfordern. Die Ursache liegt dabei in der hierdurch bedingten Abnahme der sogenannten "befestigten" oder auch "attached" Gingiva.

Für eine gute Einheilung des unter der vernähten Schleimhaut liegenden Knochenaugmentates sollte das gesamte Augmentat idealerweise vollständig von Knochenhaut überdeckt sein. Dies ist nötig, da eine Verknöcherung nur von der Knochenhaut ausgeht. Bei einer Schlitzung der Knochenhaut kommt es allerdings im Bereich der Schlitzung zum großflächigen direkten Aufliegen der Schleimhaut auf dem Augmentat. Eine knöcherne Durchwachsung und Einheilung des Augmentates ist an solchen Stellen problematisch.

Zudem kann es bei dem Schlitzen der Knochenhaut äußerst leicht zur Durchtrennung nicht nur des Periostes, sondern auch der anliegenden Schleimhaut mitsamt den versorgenden Blutgefäßen und Kapillaren kommen, sodass in diesem sehr ungünstigen Falle das Überleben der Schleimhaut in Gefahr ist. Dies hat zur Folg, dass damit ebenso das Einheilen des gesamten Augmentates häufig nicht mehr möglich ist, da ein speicheldichter Wundverschluss durch die eingeschnittene Schleimhaut unmöglich wird.

Ferner kann es auch durch Spannen der Schleimhaut über dem Knochenaugmentat beim Vernähen zu einer Unterversorgung der gespannten Schleimhaut durch Behinderung der Durchblutung (Minderperfusion) kommen. Dies führt letzten Endes zum Absterben der Mundschleimhaut, einer sogenannten Drucknekrose, was wiederum zur Perforation der Mundschleimhaut und zum Freiliegen des Knochenaugmentates in der Mundhöhle führt. Dadurch wird sich das Augmentat mit hoher Wahrscheinlichkeit infizieren und verloren gehen.

Die möglichen Komplikationen sind daher Infekte, insuffiziente Kieferkammaugmentationen, Wund- und Einheilungsstörungen der Mundschleimhaut-Periost-Schicht und ähnliches.

Es ist bereits bekannt, mit Flüssigkeit füllbare Dehnungskörper aus Kunststoff unter die Mundschleimhaut-Periost-Schicht einzubringen und aufzufüllen. Diese können beim Füllen mit Flüssigkeit ihr Volumen um maximal das Doppelte vergrößern. Auch existieren bereits mit Flüssigkeit gefüllte Dehnungskörper, die sich durch Wasseraufnahme, einem osmotischen Gradienten folgend, vergrößern, so dass hier keine Befüllung stattfinden muß. Auch hier wird lediglich eine Verdopplung des Volumens beschrieben. Hiermit können zwar einige der oben vorgebrachten Probleme behoben werden, jedoch entsteht so meist nicht genug Gewebe, um auf die oben thematisierten Verfahren komplett verzichten zu können.

Der Erfindung liegt daher die **Aufgabe** zugrunde, einen Körper anzugeben, welcher zur Gewebevermehrung beitragen kann und bei der Anwendung im menschlichen Körper möglichst wenig Komplikationen verursacht.

Diese Aufgabe wird erfindungsgemäß durch einen bioinerten Körper mit den Merkmalen des Anspruchs 1 gelöst.

Vorteilhafte Ausführungsformen der Erfindung sind in den Unteransprüchen und der Beschreibung angegeben.

Entsprechend der Erfindung weist der bioinerte Körper einen Basiskörper auf. Dieser schwillt beziehungsweise wächst bei Hydration auf das mindestens Vierfache seines Volumens an und besteht im Wesentlichen aus getrockneten und aufbereiteten Laminaria-Astteilen. Er dient zur Anwendung bei einem Verfahren zur Gewebevermehrung.

Hydration im Sinne der Erfindung kann insbesondere als mit Wasser, Gewebswasser oder Blut/Serum in Kontakt bringen verstanden werden.

Laminarien sind Braunalgen mit circumpolarer Verbreitung, die im Eulitoral der Weltmeerküsten auf der Nordhalbkugel wachsen. Mithilfe von Haftorganen, den Rhizomen, sitzen diese bis zu 5 m großen Meeresalgen auf felsigem Untergrund fest, und halten den bis zu 4 cm dicken Ast, der das mehrere Meter lange Blatt der Alge trägt. Der Ast kann auch als Stiel bezeichnet werden.

Der Befestigungsast oder -stiel der Laminaria-Alge besteht aus Polysacchariden und -galaktosiden sowie anderen polymerisierten Zuckermolekülen wie beispielsweise Phykokolloide, Alginsäure, Polyguluronsäure und Polymannuronsäure, die nicht durch Enzyme im Säugetierorganismus abgebaut werden können.

Das Material der Äste oder Stiele dieser Laminaria Algen ist der Meeresbrandung in den Uferbereichen, wo die Alge wächst, extrem gut angepasst, indem es größte Festigkeit und Flexibilität miteinander kombiniert. Dies wird erreicht, indem die Polymerketten der Pflanzen Äste sehr reich an Wasser, bis zu 80%, und dennoch sehr reißfest sind.

Werden diese Laminaria- Äste getrocknet, schrumpfen die Durchmesser der Äste auf etwa ein Fünftel ihres Ausgangsdurchmessers zusammen. Werden diese getrockneten Äste wieder in Wasser gelegt, schwellen die Äste wieder zu ihrem Ausgangsdurchmesser an.

Der Erfindung liegt die Erkenntnis zugrunde, dass die Anwendung eines Basiskörpers aus getrockneten Laminaria Ast- oder Stielteilen die aufgezeigten Probleme überwinden kann. Hierbei kann der Basiskörper bevorzugt ein stangenartiges massives Element, welches auch als Vollstange bezeichnet werden kann, sein und/oder eine stangenartige, insbesondere zylindrische, Form aufweisen. Es hat sich herausgestellt, dass die Verwendung eines stangenartigen oder stiftförmigen Körpers den Vorteil mit sich bringt, dass der bioinerte Körper stabiler ist und eine große Ausdehnung beziehungsweise Expansionsfähigkeit aufweist. Die zylindrische Form bringt Vorteile bei der Handhabung und bei der Anwendung in einem Verfahren zur Gewebevermehrung, da sie relativ einfach an die gewünschte Position im Körper eingebracht werden kann.

Der Basiskörper kann auch eine feuchtigkeitsdurchlässige Hülle aufweisen, welche mit zerkleinerten Laminaria-Astteilen gefüllt ist. Diese Laminaria-Astteilen können dabei beispielsweise gemahlen sein. Die Hülle sollte neben der Feuchtigkeitsdurchlässigkeit auch eine Dehnbarkeit aufweisen. Zum Beispiel können hierfür Dialyseschläuche verwendet werden.

Die Oberfläche des bioinerten Körpers kann beliebig gestaltet sein. Vorteilhaft ist diese glatt oder weist eine geriffelte Oberfläche auf. Abhängig vom verwendeten Einsatzbereich wird die Oberfläche entsprechend gewählt.

Eine Verbesserung der Anwendbarkeit des bioinerten Körpers ergibt sich, wenn der Basiskörper sterilisiert, insbesondere röntgensterilisiert ist. Hierbei werden weitere Komplikationen nach dem Einbringen in den Körper vermindert.

Bevorzugt werden aus den natürlichen Laminariaästen beispielsweise 10 mm auf 100 mm dicke Stangen oder Zylinder geschnitten, welche anschließend getrocknet und danach sterilisiert, insbesondere röntgensterilisiert, werden.

Vorteilhafterweise wird der bioinerte Körper zur Anwendung in einem Verfahren der Gewebevermehrung verwendet, bei dem der bioinerte Körper unter eine Schleimhaut-Periost-Schicht beziehungsweise oberhalb des Periostes innerhalb der Schleimhaut und oberhalb eines Knochens eingebracht wird. Die Schleimhaut ist bevorzugt eine Mundschleimhaut und/oder der Knochen ist bevorzugt ein Kieferknochen.

Im Folgenden wird die Anwendung des erfindungsgemäßen bioinerten Körpers weiter erläutert. Hierbei wird insbesondere auf die Vorteile der Verwendung von Laminaria als Grundmaterial für den Basiskörper eingegangen.

Der Biokörper, welcher aus getrockneten und sterilisierten stangenförmigen Laminaria-Teilen, beispielsweise in der Größe von 10 mm auf 100 mm besteht, kann durch eine einzelne Inzision unter die Mundschleimhaut-Periost-Schicht eingebracht werden. Damit liegt er zwischen dem Knochen und der Knochenhaut. Dies geschieht bevorzugt in einem Gebiet einer vorgesehenen Knochenaugmentation, eines zu deckenden Knochenaugmentates oder eines zu deckenden Kieferknochens.

Der bioinerte Körper nimmt an dieser Stelle Gewebswasser auf und schwillt beziehungsweise wächst dann meist in einer Zeit zwischen 7 und 10 Tagen auf seine ursprüngliche Größe an. Anders ausgedrückt vergrößert er sich um das mindestens Vierfache seines Volumens. Er übt somit einen kontinuierlichen, langsam zunehmenden Wachstumsanreiz auf die Mundschleimhaut-Periost-Schicht aus. Dies führt zu einer Vermehrung der Mundschleimhaut-Periost-Schicht.

Durch einen relativ kleinen Eingriff kann so ein Überschuss an Schleimhaut im Knochenaugmentatbereich generiert werden, der anschließend eine spannungsfreie Bedeckung eines Knochenaugmentats mit unverletzter Knochenhaut und einer ausreichenden Menge an Schleimhaut für einen Wundverschluss ermöglicht. Zudem ist der Wundverschluss speicheldicht und es liegt eine möglichst geringe Spannung auf der Knochenhaut und der Mundschleimhaut. Eine Schlitzung des nicht dehnbaren Periostes, ähnlich dem Prinzip einer Jalousie, zur Verlängerung der im Gegensatz dazu dehnbaren und mit dem Periost verbundenen Schleimhaut, kann somit unterbleiben, wodurch das Augmentat durch intaktes ungeschlitztes und damit unverletztes Periost abgedeckt ist, von dem ausgehend dann eine Einheilung und die gewünschte Verknöcherung des Augmentates ausgehen kann.

Ein weiterer Vorteil der Verwendung des erfindungsgemäßen bioinerten Körpers ist es, dass sich der Raum um den bioinerten Körper mit Blut, Serum und interzellulärer Flüssigkeit auffüllt. Sowohl Blut wie auch Serum und interzellulare Flüssigkeit sind mit Wachstumsfaktoren angereicherte Körperflüssigkeiten, welche die Ausdifferenzierung der mesenchymalen Stammzellen in diesem Raum stimulieren. Die mesechymalen Stammzellen sind sowohl im Blut als auch im Periost vorhanden. Hierbei ist zu berücksichtigen, dass im körpereigenen Blut beziehungsweise im Serum eine meist optimale Wachstumsfaktorzusammensetzung vorliegt. Dies verbessert das Wachstum der Mundschleimhaut.

Nach einigen, beispielsweise ca. 14 bis 21 Tagen, kann der aufgequollene, sich vergrößerte bioinerte Körper wieder entfernt werden. Vorteilhaft an der Verwendung von Laminaria als Material ist auch, dass diese inert für die Anheftung der meisten, wenn nicht aller, körpereigener Zellen sind. Dies bedeutet, dass der bioinerte Körper relativ einfach und problemlos wieder entfernt werden kann.

Nun kann ein erwünschtes Augmentatmaterial in einer beliebigen Technik in den vorbereiteten Bereich eingebracht beziehungsweise implantiert werden. Vorteilhaft ist hierbei, dass durch die vergrößerte schlauchartige Mundschleimhaut-Periost-Schicht nach dem Einbringen des Augmentats nur ein kleiner Wundverschluss nötig ist. Dies verringert die Gefahr eine Nahtdehiszenz mit unter Umständen freiliegendem und infiziertem Knochenaugmentat.

Auch bei einer offenen Methode, bei der die Schleimhaut großflächig durchtrennt wird, um das Augmentat einzubringen, bietet die Anwendung des erfindungsgemäßen bioinerten Körpers den Vorteil, durch die vermehrte Schleimhaut in ähnlicher Weise einen im Wesentlichen speicheldichten und hauptsächlich spannungsfreien Wundverschluss zu ermöglichen. Dadurch wird die Gefahr des Eintretens der oben beschriebenen Komplikationen verringert.

Durch das Verzichten auf das Schlitzen des Periosts ist das Knochenaugmentat mit einem vollständig intakten und lückenlosen Periost umschlossen, wodurch wiederum verbesserte Voraussetzungen für eine Heilung des Augmentats erreicht werden.

Durch den Jodgehalt des Laminaria-Algen-Materials, wodurch Jod durch den bioinerten Körper freigesetzt wird, hat diese Technik zudem eine lokale antibakterielle Wirkung in dem zukünftigen Implantatbereich, so dass das Risiko einer Infektion des zukünftigen Implantatbettes reduziert wird.

Mit dem erfindungsgemäßen bioinerten Körper wird somit erreicht, dass bei der Anwendung in einem Verfahren zur Gewebevermehrung auf einfache und für den Patienten relativ unproblematische Weise ausreichend Gewebe geschaffen werden kann, um für eine nachfolgende Operation beziehungsweise Behandlung zur Verfügung zu stehen.

## Patentansprüche

1. Bioinerter Körper,
mit einem Basiskörper,
welcher bei Hydration auf das mindestens Vierfache seines Volumens anwächst, welcher im Wesentlichen aus getrockneten Laminaria-Astteilen besteht, zur Anwendung in einem Verfahren der Gewebevermehrung.

2. Bioinerter Körper nach Anspruch 1,
**dadurch gekennzeichnet,**
**dass** der Basiskörper eine massive Vollstange ist.

3. Bioinerter Körper nach Anspruch 1,
**dadurch gekennzeichnet,**
**dass** der Basiskörper eine feuchtigkeitsdurchlässige Hülle aufweist, welche mit zerkleinerten Laminaria-Astteilen gefüllt ist.

4. Bioinerter Körper nach einem der Ansprüche 1 bis 3,
**dadurch gekennzeichnet,**
**dass** der Basiskörper eine stangenartige, insbesondere zylindrische, Form aufweist.

5. Bioinerter Körper nach einem der Ansprüche 1 bis 4,
**dadurch gekennzeichnet,**
**dass** der Basiskörper eine glatte oder eine geriffelte Oberfläche aufweist.

6. Bioinerter Körper nach einem der Ansprüche 1 bis 5,
**dadurch gekennzeichnet,**
**dass** der Basiskörper sterilisiert, insbesondere röntgensterilisiert, ist.

7. Bioinerter Körper nach einem der Ansprüche 1 bis 6,
zur Anwendung in einem Verfahren der Gewebevermehrung, bei dem der bioinerte Körper unter eine Schleimhaut-Periost-Schicht oder oberhalb des Periostes innerhalb der Schleimhaut und oberhalb eines Knochens eingebracht wird.

8. Bioinerter Körper nach Anspruch 7,
**dadurch gekennzeichnet,**
**dass** die Schleimhaut eine Mundschleimhaut ist und/oder dass der Knochen ein Kieferknochen ist.

9. Bioinerter Körper nach Anspruch 7 oder 8,
**dadurch gekennzeichnet,**
**dass** der Körper mittels einer Inzision einbringbar ist.

10. Bioinerter Körper nach einem der Ansprüche 1 bis 9,
**dadurch gekennzeichnet,**
**dass** die Gewebevermehrung im Gebiet eines aufzubauenden Knochens durchgeführt wird.

11. Bioinerter Körper nach einem der Ansprüche 1 bis 9,
**dadurch gekennzeichnet,**
**dass** die Gewebevermehrung im Gebiet eines zu deckenden Knochenaugmentates oder eines zu deckenden Kieferknochens liegt.
